# EUROPEAN PATENT SPECIFICATION

(11) **EP 1 492 548 B1**
(45) Date of publication and mention of the grant of the patent: **31.08.2005**
(21) Application number: 03720858.4
(22) Date of filing: 02.04.2003
(51) Int. Cl.: A61K 33/22, A61K 35/78, A61P 15/02

(54) **COMPOSITION FOR THE TOPICAL TREATMENT OF VULVOVAGINITIS AND MYCOSES**
ZUSAMMENSETZUNG ZUR TOPISCHEN BEHANDLUNG VON VULVOVAGINITIS UND MYKOSEN
COMPOSITION POUR LE TRAITEMENT LOCAL DE VULVOVAGINITE ET DE MYCOSES

(30) Priority: 08.04.2002 IT RM20020192; 05.12.2002 IT RM20020612
(43) Date of publication of application: 05.01.2005
(73) Proprietor: BERGAMON S.r.l., 00185 Roma (IT)
(72) Inventor: BATTAGLIA, Francesco, Antonino, I-00167 Roma (IT)
(74) Representative: Fiammenghi-Domenighetti, Delfina
(86) International application number: PCT/IT2003/000200
(87) International publication number: WO 2003/084552

(56) References cited:
- WO-A-98/29112
- US-A- 1 991 501
- US-A- 3 023 144
- US-A- 4 735 358
- US-A1- 2001 014 314

## Description

### Technical Field

The present invention relates to a mixture of boric acid and bergamot for use as a specific antimycotic for vulvovaginitis and for acute, chronic, and relapsing cutaneous mycoses. In particular, the invention refers to the confection of pessaries that contain two active principles, in certain percentages.

Moreover, the present invention more generally relates to a use, a dosage and exhibition pathways related to pharmaceutical forms, in orderto obtain a better utilisation, exhibition, and a lower toxicity of boric acid in association with the bergamot extract, in the eradication of mycotic vulvovaginitis and of cutaneous mycoses in general.

### Background Art

The global incidence of mycotic vaginal infections has recently noticeably increased. The Candida-induced infections are the second more common cause of vaginitis.

Among the various species of Candida, the most common one is the Candida Albicans.

In the treatment of vulvovaginitis induced by Candida Albicans, several medicines are used (nystatin, azole derivatives, etc.), but in any case a considerable number of patients is subject to frequent relapses. Moreover, infections caused by strains that are resistant to common therapies, are more and more frequent, and in the vaginal secretion of these patients it is was possible to isolate the Torulopsis glabrata, in addition to mutated forms of C. Albicans.

Conventional antimycotic treatments for the latter forms of disease, are less effective "in vitro", especially in the case of T. Glabrata, and clinical studies confirm their relative ineffectiveness.

Recent studies have.reported a percentage of success exceeding 90%, when using boric acid compounds in the therapy of vulvovaginitis induced by C. Albicans.

The bactericide and bacteriostatic properties of boric acid have been known for a long time, but their therapeutic use for mycotic infections has been proposed only after several years, following studies performed "in vitro", which demonstrated the antimycotic action on CAlbicans of boric acid at 4%.

Boric acid has successively been used thereafter, in the treatment of certain types of candidosis that were resistant to conventionally employed antimycotic therapies. However, its action directly depends on the concentration employed in its use. Very high concentrations are more effective, but their drawback is a relative loco-regional toxicity and systemic toxicity, which have limited their diffusion for what concerns the common use.

Although boric acid is effective from the therapeutic point of view, as an antiseptic agent, its safety is disputed - due to the above mentioned toxic effects - in case of accidental ingestion of a commonly used solution thereof, or in case of chronic topical exposure to its oily alcoholic preparations. Accidental ingestion may occur because the boric acid solution is colourless, odourless and indistinguishable from water if it is stored in unlabeled bottles.

Boric acid more easily penetrates the inflamed and damaged cutis or mucosa, than it penetrates the healthy cutis. In case of an aqueous solution, the absorption of boric acid by the cutis (of the vulva) may be neglected, and the amount of boric acid for unit dosis, which is used in the preparations that are generally proposed and specifically suggested by the present invention, is in any case extremely lower than the lethal dosis for an adult (which is reported to be greater than 30 g/day).

Recent clinical tests show that boric acid, by itself, presented in a vaginal pessary, is effective and does not give rise to loco-regional toxicity and/or systemic toxicity, apart from very exceptional cases. Kefler-Van-Slvke et al. have treated 400 mycotic patients with boric acid, obtaining a successful percentage of 90%. A more accurate study, which used a double-blind technique, has been performed in order to evaluate the effectiveness and safety of boric acid and nystatin. Thirty days after treatment, the patients that received boric acid showed a success ratio of 72%, while those which received nystatin showed a success ratio of 50% (p=0.02). Jovanovic et al. have compared the effectiveness of antimycotic preparations against boric acid.

Boric acid is - in 98% of all cases - effective in eliminating the symptoms, in contrast with other antimycotics, which are effective in 52% of the tests. The cited authors have however used high doses of boric acid (600 mg/day of gel for 2 weeks). For external use, as a preparation, boric acid is normally dissolved in water or vaseline at

V/V percentages that vary in the interval from 3 to 5%.

### Disclosure of Invention

The present invention provides a composition for the treatment of mycotic vulvovaginitis and mycoses in general, characterised in that it contains boric acid and extract of bergamot as active principles, the percentage of boric acid being within the interval from 1% to 3% V/V, and the percentage of the extract of bergamot being within the interval from 0.1% to 1.2% V/V.

Further embodiments of the invention are specified in the dependent claims.

The association ofboric acid and bergamot, for intra vaginal preparations, allows to use even lower amounts of boric acid, for the same effectiveness, thereby obtaining an ingenuous and useful opportunity for the eradication of mycotic infections induced by C.Albicans, tropical fungi and/or mycetes that are intrinsically resistant to common therapies.

The boric acid in association with bergamot represents a composition obtained by means of appropriate mixtures of these two substances.

### Disclosure of Preferred Embodiments

According to the present invention, it has been determined the "in-vitro" effectiveness of bergamot oil, and of two of its derivatives, that is, defurocoumarinised extract and distilled extract, against 40 strains of Candida. This evaluation has similarly been conducted with each of said compounds in association with boric acid. The 40 strains that were considered, comprised: 19 Candida albicans, 13C. glabrata, 4C. krusei, 2C. tropicalis, 1C. parapsilosis, and 1C. dubliniensis.

Tests have been conducted using the method of micro-dilutions in a broth, by adhering to the N.C.C.L.S. (National Committee for Clinical Laboratory Standard) standard, for sensitivity tests of yeast against antimycotic agents.

Briefly, for the bergamot natural oil, the defurocoumarinised extract of bergamot, and the distilled extract of bergamot, variable concentration values, from 10% down to 0.02% V/V, have been tested in a RPMI (1640 medium-Sigma Aldrich) culture medium. In the tests, which employed boric acid in association with the mentioned substances, the boric acid concentration was initially at the fixed value of3%.

The results have shown - for the bergamot natural oil - an MIC₉₀ (Minimal Inhibitory Concentration) referred to 24 hours, equal to 5%, which increased (when referred to 48 hours) to a value exceeding 10%; better values were obtained for the defurocoumarinised extract, for which the MIC₉₀ - referred to 24 hours - was 2.5%, and 5% for 48 hours; finally, the distilled extract of bergamot had the lowest MIC₉₀ values among those of the three tested substances, with values of 1.25% referred to 24 hours, and 2.5% referred to 48 hours.

The association of boric acid at 3% and each of the three substances mentioned above, gave for the latter an MIC₉₀ value - for 24 and 48 hours alike -, which was less than 0.02%, thereby emphasising the antimycotic activity of the boric acid itself. Thereafter, the activity of boric acid (taken alone) was studied, and this study has furnished values of MIC₉₀ equal to 0.094, at 24 and 48 hours alike. Later tests, performed with sub-MIC concentrations of boric acid in association with different concentrations of each of the three above mentioned substances, have shown an evident synergistic effect.

These results have shown the efficacy of the natural extract of bergamot, and above all, of the defurocoumarinised and distilled extracts - representing derivatives thereof-, against all tested strains (the sensitivity "interval" was similar among the non-Albicans strains resistant to fluconazole and the sensitive C. Albicans strains); but the essential feature resides in the possibility of using small concentrations of bergamot in association with small concentrations of boric acid, in order to reduce possible toxic effects thereof without reducing its efficacy at all.

Preferably, the above described chemical compound is prepared in the form of vaginal pessaries.

In the above description, the use of the defurocoumarinised or distilled extracts of bergamot in association with boric acid has been disclosed exclusively for gynaecologie applications, and specifically when these substances are presented as a vaginal pessary for the eradication of mycotic infections induced by C. Albicans, tropical mycetes and/or mycetes which are intrinsically resistant to the usual therapies. The present invention, according to a further aspect thereof, has shown that even generic mycoses may be cured by means of these active principles combined with each other. Obviously, althoughvaginal pessaries are very handy, they do not allow any dosage or application flexibility, since they do not provide for any other topical application nor for the splitting up (subdivision) of the pessary, which necessarily must maintain its rounded shape.

Therefore, a further aspect of the present invention provides for substantial improvements in the treatment of mycoses in general, so as to broaden the exhibition forms and the utilisation forms (means) of the aforesaid mixture, thereby modifying at the same time the respective dosages in order to obtain a versatile product, which may be more easily dosed and which, consequently, is less toxic.

Obviously, this enables a more versatile use and permits a more accurate and more appropriate dosage, according to the virulence, the extension and the location of the mycosis, which must no more be localised exclusively in the vagina for allowing a treatment by means of the above mentioned compounds, but may- instead - be treated even if it affects any region whatever of the skin or of the externally accessible mucosae.

The said product may easily be prepared in the form of emulsions, pomades, ointments, creams, pastes, gels and foams, as exhibition means of the same compound, so as to provide new and better means for administering the above specified active principles even externally of the vaginal region, and so as to extend their use to the whole topical sphere.

These new pharmaceutical means for topical administration, allow - besides a better handiness -, also a reduction in the dosis of the active principles contained in the medicine, which, for boric acid, may signify a decrease of up to 66% with respect to the above 3 %, reaching a percentage which starting from 1% does not - in any case - exceed 3%. In particular, the toxicity is reduced together with side effects and sensitisation effects.

Also, the bergamot oil contained in these new pharmaceutical administration means, may be present in doses that may vary from 0.1 to 1.2%, instead of the sole preferred 0.2% concentration that has been used above for the mycotic vulvovaginitis. By broadening in this manner the upper and lower limits of the useful ratio of said active principle, one prevents as much as possible any shortcomings due to toxicity and acute sensitisation.

## Claims

1. A therapeutic compound for the treatment of mycotic vulvovaginitis and mycoses in general, **characterised in that** it contains boric acid and extract of bergamot as active principles, the percentage of boric acid being within the interval from 1% to 3% V/V, and the percentage of the extract of bergamot being within the interval from 0.1% to 1.2% V/V.

2. A compound according to claim 1, wherein the extract of bergamot is bergamot oil, or the defurocoumarinised bergamot derivative (Citrus bergamia), or the distilled bergamot extract.

3. A. compound according to claim 2, that is specifically used for mycotic vulvovaginitis, wherein the boric acid percentage is about 3% V/V , and the percentage of bergamot oil, or of the defurocoumarinised derivative of bergamot, or of the distilled bergamot extract, is about 0.2%.

4. A compound according to claims 1 and 2, wherein the said compound is associated with dyes, dispersion agents, excipients, emulsifiers, and friction reducing agents, to form creams, ointments, emulsions, pomades, pastes, gels, and foams, that may be very easily handled and locally dosed on the whole surface of the cutis affected by an acute, chronic or relapsing mycosis , or else by a mycotic wlvovaginitis, thereby improving the administration, broadening the application field and reducing side effects.

5. A compound according to claim 3, presented in the form of a vaginal pessary, and containing excipients, dispersion agents, emulsifiers and/or friction reducing agents, such as to allow a correct and homogeneous dilution of the two active principles therein, to obtain good handling properties, and a satisfactory consistence at ambient temperature, together with a satisfactory stability of the adequately sealed product.

## Patentansprüche

1. Therapeutische Zusammensetzung zur Behandlung der mykotischen Vulvovaginitis, **dadurch gekennzeichnet dass** sie Borsäure und Extrakt der Bergamotte als aktive Stoffe enthält, wobei der Prozentinhalt an Borsäure im Intervall zwischen 1% und 3% (Volumenprozent) und der Prozentinhalt an Bergamotte im Intervall 0.1 bis 1.2 % (Volumenprozent) liegen.

2. Zusammensetzung nach Anspruch 1, wobei der Bergamottextrakt ein Bergamottöl oder das defurocumarinisierte Bergamottderivat (Citrus bergamia), oder destillierter Begamottextrakt ist.

3. Zusammensetzung nach Anspruch 2, welche spezifisch gegen die Vulvovaginits benutzt wird, wobei der Prozentinhalt an Borsäure ca. 3% (Volumen) beträgt und der Prozentinhalt an Bergamottöl oder an defurocumarinisiertem Bergamottderivat oder an destilliertem Bergamottextrakt ca. 0.2% betragen.

4. Zusammensetzung nach Anspruch 1 und 2, wobei diese Zusammensetzung mit Farbstoffen, Dispergiermitteln, Trägerstoffen, Emulgiermitteln, und Reibung reduzierenden Mitteln assoziiert ist, um Cremen, Heilsalben, Emulsionen, Salben, Gele, und Schäume zu erzeugen, welche leicht zu handhaben sind und lokal dosiert werden können über die ganze Hautoberfläche die betroffen ist von einer akuten, chronischen, oder rückfälligen Mykose, bzw. von einer mykotischen Vulvovaginitis, so dass auf diese Weise die Verabreichung verbessert, das Anwendungsfeld erweitert, und die Nebenwirkungen reduziert werden.

5. Zusammensetzung nach Anspruch 3, welche in der Form eines Arzneizäpfchens vorliegt, und Trägerstoffe, Dispergiermittel, Emulgiermittel und/oder Reibung reduzierende Mittel enthält, um eine genaue und homogene Verteilung der beiden Aktivstoffen darin zu bewirken, und dabei gute Handhabungseigenschaften und eine befriedigende Dichtigkeit bei Normaltemperatur zu erreichen, und dazu eine befriedigende Stabilität des mit angemessener Dichtigkeit verpackten Produktes.

## Revendications

1. Composition thérapeutique pour le traitement de vulvovaginites mycotiques et mycoses en général, **caractérisée** du fait de conteinir acide borique et extrait du bergamotier comme principes actives, le pourcentage du acide borique est compris entre 1' 1 % et le 3% V/V, et le pourcentage de l'extrait du bergamotier est compris entre le 0,1% et le 1,2% V/V.

2. Composition selon la revendication 1, dont l'extrait du bergamotier est huile du bergamotier, ou dérivé defurocoumarinizé du bergamotier (Citrus bergamia), ou extrait distillé du bergamotier.

3. Composition selon la revendication 2, dont l'utilisation est spécifique pour les vulvovaginites mycotiques, dans lesquelles le pourcentage du acide borique est presque le 3% V/V, et le pourcentage de 1' huile du bergamotier, ou du dérivé defurocoumarinizé du bergamotier, ou extrait distillé du bergamotier, est presque le 0,2%.

4. Composition selon les revendications 1 et 2, dont le composé est associé avec colorants, additifs dispersants, excipients, émulsifiants, agents lubrifiants, pour former des crèmes, des ounguents, des émulsifiants, des pommades, des pàtes, des gels et écumes qui sont très simples à manier et qui sont dosés localement sur la surface de la peau atteint par des mycoses acutes, chroniques ou récidivantes, ou par des vulvovaginites mycotiques, améliorant l'administration, étendant le domaine d'application en réducant les effects collatéraux.

5. Composition selon la revendication 3, présentée en forme de ovule vaginal, et contenant des excipients, des agents dispersants, des émulsifiants, et/ou des agents lubrifiants, pour permettre une correcte et homogène diluition des deux principes actives, pour obtenir des bonnes proprietées de maniement et une satisfaisante consistence à la temperature ambiante, avec une satisfaisante stabilité du produit cacheté.
